# EUROPEAN PATENT SPECIFICATION

(11) **EP 3 107 941 B1**
(45) Date of publication and mention of the grant of the patent: **16.07.2025**
(21) Application number: 15715502.9
(22) Date of filing: 19.02.2015
(51) Int. Cl.: C08B 37/08, A61K 31/722, A61K 47/36, A61K 38/06, C07K 5/02, A61L 27/20, C08L 5/08, C09D 105/08, A61P 17/00

(54) **METHOD FOR SYNTHESIS OF A BIOPOLYMER DERIVATIVE, A BIOPOLYMER DERIVATIVE AND ITS USE**
VERFAHREN ZUR SYNTHESE EINES BIOPOLYMERDERIVATS, BIOPOLYMERDERIVAT UND DESSEN VERWENDUNG
PROCÉDÉ DE SYNTHÈSE D'UN DÉRIVÉ DE BIOPOLYMÈRE, DÉRIVÉ DE BIOPOLYMÈRE ET SON UTILISATION

(30) Priority: 19.02.2014 PL 40725714; 19.02.2014 PL 40725814
(43) Date of publication of application: 28.12.2016
(73) Proprietor: Instytut Biochemii I Biofizyki Polskiej Akademii Nauk, 02-106 Warszawa (PL)
(72) Inventor: BAL, Wojciech, PL-05-509 Mysiadlo (Piaseczno) (PL); ZAWISZA, Izabela, PL-28-100 Busko Zdrój (PL); FRACZYK, Tomasz, PL-23-804 Stromiec (PL)
(74) Representative: Swiecicki, Krzysztof
(86) International application number: PCT/PL2015/000023
(87) International publication number: WO 2015/126269

(56) References cited:
- WO-A1-2012/171125
- WO-A1-2014/021726
- WO-A1-92/09636
- CA-A1- 2 126 132
- CN-A- 104 012 839
- CN-A- 104 387 503
- CN-B- 102 133 433
- US-A1- 2007 299 034
- ZARECKI ADAM P. ET AL: "Microwave-Assisted Catalytic Method for a Green Synthesis of Amides Directly from Amines and Carboxylic Acids", MOLECULES, vol. 25, no. 8, 1 April 2020 (2020-04-01), DE, pages 1761, XP093034390, ISSN: 1433-1373, DOI: 10.3390/molecules25081761
- LI JUNJIE ET AL: "A chitosan-glutathione based injectable hydrogel for suppression of oxidative stress damage in cardiomyoc", BIOMATERIALS, vol. 34, no. 36, December 2013 (2013-12-01), pages 9071 - 9081, XP028714771, ISSN: 0142-9612, DOI: 10.1016/J.BIOMATERIALS.2013.08.031
- KRUM KAFEDJIISKI ET AL: "Synthesis and in Vitro Evaluation of a Novel Chitosan-Glutathione Conjugate", PHARMACEUTICAL RESEARCH, KLUWER ACADEMIC PUBLISHERS-PLENUM PUBLISHERS, NL, vol. 22, no. 9, 1 September 2005 (2005-09-01), pages 1480 - 1488, XP019370937, ISSN: 1573-904X, [retrieved on 20050824], DOI: 10.1007/S11095-005-6248-6
- SEUNG HYUN KOO ET AL: "Preparation, Characteristics, and Stability of Glutathione-Loaded Nanoparticles", JOURNAL OF AGRICULTURAL AND FOOD CHEMISTRY, vol. 59, no. 20, 26 October 2011 (2011-10-26), pages 11264 - 11269, XP055192044, ISSN: 0021-8561, DOI: 10.1021/jf2024648

## Description

### Field of the invention

The invention relates to chitosan derivative, wherein said chitosan is modified with glutathione with the formation of a peptide bond, to form chitathione, a compound having peptide bonds between amine groups of units of poly(2-deoxy-2-aminoglucose) and available carboxyl groups of glutathione, for use in preventing symptoms of skin allergy caused by the contact with metals, especially heavy metals, in particular nickel. The invention relates also to a cosmetic composition containing a new chitosan derivative for use in preventing symptoms of skin allergy caused by heavy metals.

### Background of the invention

Water insoluble polymers of biological origin, e.g. polysaccharides, such as chitosan, comprise compounds containing active amine or carboxyl groups susceptible to formation of peptide bond.

Chitosan is a linear polysaccharide, composed of β-(1-4)-linked D-glucosamine and N-acetyl-D-glucosamine units. It can be produced from animal or fungal chitin and has multiple applications e.g. in the cosmetic industry and medicine.

In the prior art classical peptide synthesis in the solid phase has been used. Resins for peptide synthesis according to the Fmoc/SPPS protocol, conditions for attaching the first amino acid to the resin and for elongation of the peptide by means of generating the peptide bond are known in the prior art.

CA2126132A1 discloses a method of peptide synthesis on chilosan. However classical synthesis and an additional binding molecule between the amino group of chitosan and the first amino acid of the peptide are used.

There was no disclosure in the prior art of a use of fully biodegradable biopolymer, such as chitosan, as resin for attaching modifiers with formation of a direct peptide bond between chitosan and the modifier molecule. Under conditions applicable for classical peptide synthesis the reaction takes a long time and gives no satisfactory yield.

EP1512773 discloses incorporation of biologically active components into chitosan layer(s) while coating metal-made medical devices. However, in this method biomolecules are added as a separate coating layer, without forming specific covalent bonds.

Methods derived from classical peptide synthesis in the solid phase for obtaining chitosan derivatives involve many inconveniences, e.g. low yields and long reaction times.

Li Junjie et al., Biomaterials, 2013 Dec; 34 (36): 9071-81. Doi: 10.1016/j.biomaterials.2013.08.031. Epub 2013 Aug 31, describe the method of preparing chitosan and glutathione conjugates and the product obtained by this method. The method of preparing chitosan and glutathione conjugates is based on a reaction carried out for 6 hours, using EDC. The conjugates are used as a reactive oxygen scavenger in myocardial infarction. As results from the used method for obtaining the chitosan derivative, this document relates in fact to the chitosan chloride derivatives but not a chitosan derivative, wherein said chitosan is modified with glutathione with the formation of a peptide bond. The chloride per se and its derivatives are known as skin irritating agent. Thus those derivatives are of no use according to the application on skin. The method leading to chloride derivative is of no use for the present invention.

Kafedjiiski Krum et al. Pharm Res. 2005 Sep; 22 (9): 1 480-8. Epub 2005 Aug 24 discloses a chitosan-glutathione derivative, which is also obtained with the use of completely different method and is different derivative then disclosed in the pending application. As detailed in Table I of Kafedjiiski K. et al. the method described therein yields a derivative, which contains a mixture of GSH thiol and disulfide, with the excess of the latter. The method comprises use of EDAC and NHS reagents and 7 hours are necessary to complete the reaction. Use of such reagents in the method leads to the formation of extremely harmful by-products, so it is evident that the final derivative also comprises such harmful by-products. Moreover, the yield of their process is very low, providing less than 80 mg GSH per 1 g of chitosan, which is contaminated with ca. 120 mg of GSSG. Thus, it is clear for the person skilled in the art, that Kafedjiiski K. et al. discloses different "chitosan-glutathione derivative" then the chitosan derivative of the present invention. Contrary to the present application is also use of the derivative, since this document is focusing on maintaining the GSH permeation enhancing properties. This is in direct opposition to the idea of the present invention, where the derivative is used as a barrier, in order to stop penetration of the metal ions to the skin or to entrap the ions in purification of the wastes.

The method according to Koo Scung Hyun et al. J. Agric. Food Chem., 2011, 59 (20), pp. 11264-11269 is similar to that described in Kafedjiiski K. et al., but the reaction takes even more time i.e. 15 hours. Thus, the obtained GSII-chitosan derivative must be analogous to the derivative of Kafedjiiski K. et al. and the final product is also contaminated. In fact Koo et al. focuses on chitosan-glutathione nanoparticles as a system for GSH delivery to the living organisms and to maintain the GSH antioxidant activity based on its radical scavenging activity. Thus the use of the method for obtaining GSH-chitosan derivative is completely out of the field of the present invention. Similarly, CN 102 133 433 relates also to the chitosan-glutathione derivative obtained with the use of EDC and NHS with reaction lasting 5 hours.

Even though CA 2 126 132 A1 relates to the method with the use of HBTU, HOBT and DIPEA, but does not relate to use of microwaves for the reaction. The method according to CA 2 126 132 A1 comprises attaching a bifunctional linker molecule to the free amino groups of the chitosan, prior to the step of attaching the first amino acid. Thus, CA 2 126 132 A1 relates to the different method then the present invention.

Use of the microwaves for N-alkylation was described in WO 2012/171125. However this document does not relate to peptide bond formation and not for obtaining a glutathione-chitosan derivative. The method according to WO 2012/171125 differs from the method according to the present invention by the reagents and conditions used, such as temperature 130⁰C and 1600 Watts vs. 75°C according to the method of the pending application. Even more important is that according to the method described in WO2012/171125 the same yield is obtained with and without use of microwaves. In result the skilled person would not see any advantage with using microwaves for N-alkylation of chitosan, and would even not try to use microwaves in peptide bond formation. A person skilled in the art will easily recognize that conditions needed for the reaction in WO2012/171125 are analogous to those used for destruction of peptide bonds in biochemical practice.

None of the document of the prior art discloses free of toxic impurities chitosan derivatives with glutathione and a method for obtaining thereof. Also, there is no document disclosing or suggesting use of microwaves in combination with either DCC and HOPfp or HBTU, HOBT and DIPEA activators in a mild condition such as temperature 75°C or lower and time reaction 30 minutes or shorter for obtaining a peptide bond between chitosan and another molecule.

Use of chitosan for treatment of skin irritation caused by various factors is known from the prior art. RU 2357738C2 describes the use of dermatological preparation containing 1% chitosan hydrochloride for prevention and treatment of platinosis. WO 9209636 (A1) relates to a method of protecting the skin from contact with an allergen or toxin comprising applying to the skin of a subject sensitized to said allergenic agent, prior to contact with said skin, a polysaccharide selected from chitosan polymers and chitosan derivatives. This document neither mentions an antigen being a metal, nor a possibility of application of chitosan derivatives, in which their amino group would be engaged in a peptide bond. Further, EP1512773 discloses chitosan coating of metal-made medical devices to make them biocompatible and to prevent irritation of the organism with metal ions derived from these devices.

The most frequent nickel allergy manifestation is contact dermatitis caused by response related to Th lymphocytes (type IV hypersensitivity). Nickel is one of the most frequent allergens. More than 15% of population of developed European countries (e.g. Germany, United Kingdom, Italy) suffers nickel allergy. In Poland 30% of 16-17 year old girls suffer from nickel allergy [Br. J. Dermatol. 2013, 169: 854-858; Contact Dermatitis 2011, 64: 121-125]. The importance of the problem is stressed by the fact that the EU Directive 94/27/EC regulates the allowed amount of nickel released from jewelry and common use objects.

Therefore, there is an urgent need for the development of effective protection against allergy to metals, especially allergy to heavy metals, in particular to nickel.

The invention disclosed herein solves the problem of a synthetic method that provides sufficient efficacy for industrial application of the obtained derivatives, as well as solves the problem of a delivery on an industrial scale of active molecules based on safe, nontoxic, biodegradable compounds, such as chitosan.

The subject of the invention is defined in the attached set of claims.

### Disclosure of the invention

Herein disclosed is an insoluble polymer derivative containing reactive amine or carboxyl groups, modified by a modifier molecule with the use of formation of a peptide bond. Preferably, the polymer is a poly/oligosaccharide, preferably chitosan. The peptide bond is present between the amine group of a subunit of poly(2-deoxy-2-aminoglucose) and one of the available carboxyl groups of a modifier molecule. Preferably, the modifier molecule containing the carboxyl group is selected from carboxylic acids, amino acids, amino acid analogues, dipeptides, tripeptides, tetrapeptides, longer peptides, peptidomimetics, proteins and protein mimetics or any mixture thereof.

Disclosed is also a method of synthesis of a derivative of an insoluble polymer, in which peptide bonds are formed between the amine or carboxyl groups of units of the biopolymer containing reactive amine or carboxyl groups, and the available carboxyl or amine groups of the modifier molecules being attached.

The method as disclosed comprises
a) reacting the modifier molecule with Fmoc-Cl in a suitable solvent, preferably dioxane;
b) dissolving the Fmoc-modifier molecule in a solvent, preferably DMF, and reacting it with a biopolymer in the presence of HBTU, HOBt and DIPEA;
c) removing the Fmoc protecting group from the modifier molecule and
d) suspending the biopolymer with the modifier molecule attached to it in distilled water and lyophilizing.

The disclosed method comprises forming a peptide bond between the amine group of a unit of poly(2-deoxy-2-aminoglucose) wherein the peptide bond between the polymer and the modifier molecule is formed in the field of microwaves.

The step of obtaining the Fmoc protected peptide molecule can be omitted.

As disclosed, the preferred method comprises:
a) processing the biopolymer in a suitable solvent with microwaves,
b) adding activators to activate the functional group of the biopolymer,
c) adding the unprotected modifier molecule,
d) processing the reaction mixture with microwaves,
e) washing and lyophilizing the obtained modified biopolymer.

DCC and HOPfp or HBTU, HOBT and DIPEA are used as preferred activators. Preferably, the processing with microwaves is continued for 1-30 minutes. Nevertheless, even longer times are also included in the disclosure, as far as they lead to the peptide hond formation.

The method for synthesis comprises chitosan as the biopolymer and the modifier molecule is selected from at least one of carboxylic acids, amino acids, amino acid analogues, dipeptides, tripeptides, tetrapeptides, longer peptides, peptidomimetics, proteins or protein mimetics, preferably the tripeptide molecule is glutathione.

Disclosed is also a cosmetic or pharmaceutic composition containing the biopolymer derivative according to the invention. The composition is in a form of ointment, cream, lotion. The composition is used in medicine, medicinal products or cosmetic products.

Disclosed is also a composition for use in preventing symptoms of allergy caused by heavy metals, preferably palladium, cobalt, chromium and gold, most preferably nickel.

Preferably, the composition contains an additional biopolymer, preferably a biopolymer being a metal binding protein.

Moreover, disclosed is a chitosan derivative for use in preventing symptoms of skin allergy caused by contact with metals, preferably with heavy metals, most preferably with nickel.

Disclosed is also a matrix for attaching modifier molecules, containing the biopolymer derivative according to the invention.

Disclosed is the use of a chitosan derivative and/or chitosan for preventing symptoms of allergy caused by heavy metals, preferably for preventing symptoms of allergy caused by nickel. Preferably the composition contains an additional biopolymer, preferably a biopolymer being a metal binding protein.

Further, disclosed is a method for purification of industrial and domestic waste wherein the biopolymer derivative according to the invention is contacted with said waste to entrap the pollutants and said biopolymer derivative with entrapped pollutant is resolved. Disclosed is also a method for recovery of metals wherein the material containing metal ion is contacted with the biopolymer derivative according to the invention. Thus, disclosed is the use of a biopolymer derivative according to the invention for purification of industrial and domestic waste and/or recovery of metals.

### Detailed description of the invention

The term "insoluble biopolymer" denotes all chemical compounds of biological origin consisting of units (mers), these compounds contain active amine or carboxyl groups susceptible for the formation of peptide bonds. These compounds are principally water insoluble. Cellulose and chitosan are examples of insoluble biopolymers.

The term "modifier molecule" relates to the molecule that is able to form a peptide bond with chitosan or other biopolymer molecule having carboxyl or amine moieties.

The term "peptidomimetics" or "protein mimetics" relates to a modification or cyclization of linear peptides or proteins. The examples of peptidomimetics comprise amide bond surrogates, peptidosulfonamides, phosphonopeptides, oligourcas, depsides, depsipeptides, and peptidoids.

Chitosan is practically insoluble in water and organic solvents suitable for peptide synthesis, but it is chemically active, thus enabling its use as matrix for attaching organic molecules, e.g. peptides.

Disclosed is the modification of chitosan with molecules containing carboxylic groups suitable for the formation of peptide bonds under classical conditions of formation of peptide bonds and in the field of microwaves. Examples of such molecules include carboxylic acids, amino acids, amino acid analogues, dipeptides, tripeptides, tetrapeptides, longer peptides, peptidomimetics, proteins or protein mimetics or any mixture thereof.

By means of selection of an appropriate modifier molecule it is possible to obtain desired properties of a biopolymer being modified. For example, by attaching the molecule of glutathione - a tripeptide forming complexes with metal ions- to a monomeric unit of the polymer, it is possible to significantly increase the ability of chitosan to chelate metal ions. The selection of appropriate modifiers may affect a number of properties of the biopolymer, such as its solubility, the pH value after its suspension in water, as well as its fungicidal and bactericidal properties.

The method of synthesis of chitosan modified with glutathione according to the disclosure employs the commercially available chitosan or another insoluble biopolymer characterized by the presence of reactive amine or carboxyl groups and the modifier molecule, e.g. peptide.

In the method of synthesis of chitosan modified with glutathione according to the disclosure, peptide bonds are formed between the amine groups of poly(2 deoxy-2-aminoglucose) molecule, where 2-deoxy-2-aminoglucose is a monomer forming the structure of chitosan, and one of available carboxyl groups of glutathione, according to the synthetic strategy designed for the purpose of this invention and under optimized conditions. Fmoc-glutathione is used in the reaction, with the amine function group protected according to the procedure described in Example 1. The procedure applied for the formation of the peptide bond is described in Example 2.

The coupling reaction in the field of microwaves was used in the method as disclosed, to synthesize new derivatives. Microwaves additionally activate amine groups of the biopolymer, and also facilitate access of modifier molecules to function groups of the polymer by influencing its structure.

The microwaves play an essential role in the method of peptide bond formation as disclosed. The generation of the peptide bond in the field of microwaves significantly increased the reaction yield (by a factor of 100, from 0.3% to 30%) and at the same time reduced the reaction time (from 450 minutes to 20 minutes). It also helps avoid a difficult and expensive step of protecting the amine group of the modifier molecule.

In the first step of reaction the biopolymer is strongly activated in the field of microwaves, which makes its function groups much more active than the function groups of the modifier. Next, the coupling of the modifier molecules with appropriate function groups of the biopolymer and attaching the modifier molecule to the matrix is conducted. The formation of di- tri- or even polymeric products composed of molecules of unprotected modifier is a possible side reaction. Such reaction was prevented by previous activation of the biopolymer, which privileged the reaction of the biopolymer with the modifier molecule. This activation made it possible to avoid protecting the amine group of the modifier molecule with fluorenylmetoxycarbonyl chloride, thus eliminating two reaction steps: protecting the amine group and removing the protection after the coupling reaction. This results in a significant reduction of reaction cost and also provides a green chemistry aspect to the invention. The environmentally hazardous fluorenylmetoxycarbonyl chloride is not used any more in the reaction, which is conducted with the use of a biocompatible polymer, biodegradable modifiers, popular activators and volatile solvents.

The biopolymer - chitosan is a nontoxic compound, and thus its use, even on an industrial scale, does not evoke environmental pollution. Biocompatibility is an important property of this polymer. Its further advantages are high adhesivity and absorptivity, high chemical reactivity and ability to chelate metal ions, resulting from the presence of an amine group in each of its units (mers). In an aqueous environment it interacts with metal ions forming coordination bonds. Due to its ability to assume many spatial conformations, this polymer can also enclose metal ions within its structure. A clear advantage of chitosan is also its property to serve as a nontoxic and envirommentally friendly matrix for attaching modifier molecules.

The manipulation of properties of a biopolymer, in particular those regarding the increase of metal binding strength and/or selectivity opens up a wide field of various applications of modified biopolymers, c.g. in cosmetic industry, pharmacy and environmental protection.

Disclosed herein is attaching glutathione to chitosan. Preferably, the synthesis is performed with the use of field of microwaves. An appropriate reaction vessel and a microwave reactor can be used for this purpose. Chitosan is processed with microwaves for the period of time sufficient for the activation of function groups of the polymer, preferably for 1-30 minutes, at 25-100 °C and power P-10-50 W.

Preferably, the function groups of chitosan are activated with DCC and I-IOPfp. Also preferably, activation of function groups is followed by contacting chitosan with glutathione which is not protected by Fmoc and the resulting reaction mixture is again processed with microwaves for a required period of time in an appropriate temperature. Chitosan is processed with microwaves at least twice. Preferably, the exposure to microwaves lasts for 1-20 minutes, power is in the range of 10-25 W and the reaction occurs at 20-70 °C. The product can be recovered according to standard procedures, such as centrifugation and lyophilization.

As disclosed, attaching of glutathione to chitosan in the field of microwaves is performed with the use of HBTU, HOBT and DIPEA. The time of exposure, power and temperature are selected to activate the chitosan function groups.

The product present in a form of suspension is centrifuged and the supernatant is decanted. The obtained modified biopolymer is washed at least once, preferably two or three times with fresh portions of DMF and centrifuged. Preferably, this procedure is repeated with methylene chloride.

Attaching of bacitracin or ticarcillin to chitosan in the field of microwaves is performed with the use of HBTU, HOBT and DIPEA.

Disclosed herein, modified chitosan captures metal ions, preferably of nickel or other heavy metals.

### Brief description of drawings.

The method as disclosed is explained on the basis of the specific examples in more detail on Figures wherein:
**Figure 1** presents the scheme of reaction of chitosan modification with a modifier molecule under the conditions of peptide boud formation.
**Figure 2** presents the ESI-MS spectrum of 9-fluorenylmetoxycarbonyl-glutathione obtained in the reaction of glutathione coupling with Fmoc-Cl.
**Figure 3** presents a comparison of behavior of commercially available chitosan (A) and glutathione-modified chitosan (A[GSH]) in contact with a 50 mM solution of nickel(II) chloride. The precipitate in test tube marked (A) is brown and the precipitate in test tube marked (A[GSH]) is green. Nickel complexes with unmodified chitosan are green, while nickel complexes with glutathione-modified chitosan are brown.
**Figure 4** presents a graph illustrating a comparison of nickel binding potency of commercial chitosan (A) and glutathione-modified chitosan (A[GSH]).

### EXAMPLES

Examples are provided herein below. However, the disclosed and claimed invention is to be understood to not be limited in its application to the specific experimentation, results and laboratory procedures. Rather, the Examples are simply provided as one of various embodiments and are meant to be exemplary, not exhaustive.

### Example 1 (reference)

### Synthesis of 9-fluorenylmetoxycarbonyl-glutathione.

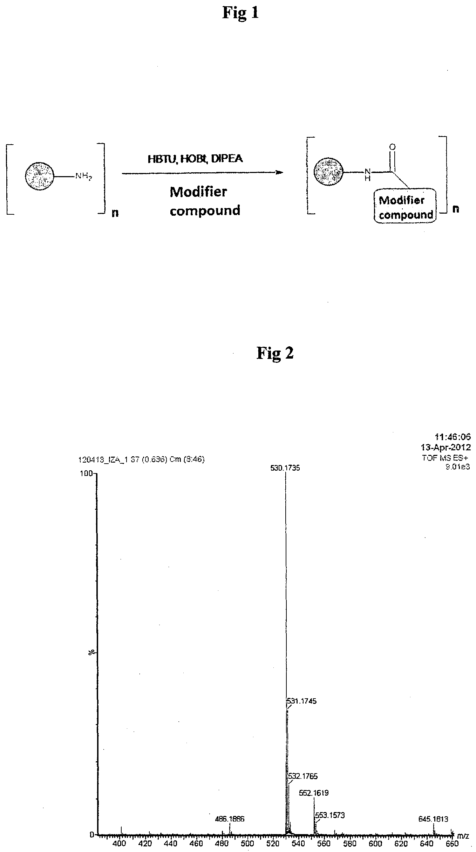

In a 200 ml three-necked flask 3 g of glutathione (10 mmoles) was dissolved in a mixture of 26 ml of dioxane and 68 ml of 10% NaCO₃ under anaerobic conditions. The flask fitted with a dropping funnel, stirring magnet, argon balloon and a bubbler was mounted over a magnetic stirrer. 2.71 g of Fmoc-Cl (10.5 mmoles) was dissolved in 26 ml of dioxane and added dropwise slowly over 15 minutes. The reaction was kept in the ice bath during addition. Then, the ice bath was removed. The reaction was allowed to proceed for 10 hours under argon, while monitoring its progress by ESI-MS. Next, the solution was acidified to pH≈3. The precipitate formed was separated on a Schott funnel. The remaining solution was evaporated until a significant amount of precipitate formed. This precipitate was separated on a Schott funnel and washed with distilled water. Fmoc-glutathione was obtained, having molecular mass 529.17 g/mole (Fig. 2).

### Example 2

### Attaching of glutathione to chitosan

0.68 g of chitosan was placed in a reaction vessel for solid state peptide synthesis. 3 g of Fmoc-glutathione (3 mol equivalents) was dissolved in 20 ml of DMF. To this solution 2.14 g (3 mol equivalents) of HBTU, 1.29 g (3 mol equivalents) of HOBt and 1.98 ml (6 mol equivalents) of DIPEA were added. The reagents were mixed together and added to the reaction vessel containing chitosan. The mixture was allowed to react for 2.5 hours on a laboratory shaker. This procedure was repeated three times. Next, the solution was filtered off and the remaining biopolymer was washed three times with DMF. In order to remove the Fmoc protecting group from glutathione, a 20% solution of piperidine in DMF was added twice, followed by shaking for 20 minutes. Following the Fmoc group removal, the biopolymer was washed three times with DMF. The DMF solution was sucked up and the biopolymer with glutathione was suspended in distilled water and lyophilized. The reaction yield determined by elemental analysis Y<0.3%.

### Example 3

### Attaching of glutathione to chitosan in the field of microwaves according to method 1 with the use of DCC and HOPfp.

To a reaction vessel for solid state peptide synthesis 2.27 g of chitosan suspended in 5 ml of 2:1 DMF:H₂O mixture was added and processed with microwaves (t=5 minutes, P=25 W, T=75 °C). 0.613 g of HOPfp and 0.687 g of DCC were dissolved in 5 ml of 2:1 DMF:H₂O mixture and added to the reaction vessel. The resulting mixture of chitosan with the activators was processed with microwaves (t=5 minutes, P=25 W, T=75 °C), which activated the function groups of the polymer. Next, 0.568 g of glutathione (free molecule, not protected with Fmoc) in 5 ml of DMF was added to the vessel and subjected twice to the microwaves (t=5 minutes, P=12 W, T<50 °C). The suspension was added to a centrifugation vessel and centrifuged. The supernatant was decanted. The obtained modified biopolymer was suspended three times in fresh portions of DMF, and then centrifuged and decanted. This procedure was repeated with the use of methylene chloride. After these three washes with methylene chloride the precipitation was frozen in liquid nitrogen and lyophilized. The lyophilized precipitate was washed three times with distilled water and lyophilized again. Elemental analysis revealed the presence of sulfur, and therefore the presence of glutathione attached to the polymer. The reaction yield determined by elemental analysis Y = 23%.

### Example 4

### Attaching of glutathione to chitosan in the field of microwaves according to method 2 with the use of HBTU, HOBT and DIPEA.

To a reaction vessel for solid state peptide synthesis 2.27 g of chitosan suspended in 5 ml of DMF was added and subjected to the action of microwaves (t=5 minutes, P=25 W, T=75 °C). 2.14 g HBTU, 1.29 g HOBT and 1.98 ml DIPEA in 5 ml of DMF were added to the reaction vessel. The resulting mixture of chitosan with the activators was subjected to the action of microwaves (t=5 minutes, P=25 W, T=75 °C), which activated the function groups of the polymer. Next, 0.568 g of glutathione (free molecule, not protected with Fmoc) in 5 ml of DMF was added to the vessel and subjected twice to the action of microwaves (t=5 minutes, P=12 W, T<50 °C). The suspension was added to a centrifugation vessel and centrifuged. The supernatant was decanted. The obtained modified biopolymer was suspended three times in fresh portions of DMF, and then centrifuged and decanted. This procedure was repeated with the use of methylene chloride. After these three washes with methylene chloride the precipitation was frozen in liquid nitrogen and lyophilized. The lyophilized precipitate was washed three times with distilled water and lyophilized again. The reaction yield determined by elemental analysis Y = 30%.

### Example 5 (reference)

### Attaching of bacitracin to chitosan in the field of microwaves. according to method 2 with the use of HBTU, HOBT and DIPEA.

2.27 g of chitosan suspended in 5 ml of DMF was placed in a reaction vessel for solid state peptide synthesis and subjected to the action of microwaves (t=5 minutes, P=25 W, T=75 °C). 2.14 g HBTU, 1.29 g HOBT and 1.98 ml DIPEA in 5 ml of DMF was added to the reaction vessel, The resulting mixture of chitosan with the activators was processed with microwaves (t=5 minutes, P=25 W, T=75 °C), thus activating the function groups of the polymer. Next, 2.630 g of bacitracin dissolved in 5 ml of 1:1 DMF:H₂O mixture was added to the vessel and subjected twice to the action of microwaves (t=5 minutes, P=12 W, T<50 °C). The suspension was transferred to a centrifugation vessel and centrifuged. The supernatant was decanted. The obtained modified biopolymer was suspended three times in fresh portions of DMF, and then centrifuged and decanted. This procedure was repeated with the use of methylene chloride. After these three washes with methylene chloride the precipitation was frozen in liquid nitrogen and lyophilized. The lyophilized precipitate was washed three times with distilled water and lyophilized again. The reaction yield determined by elemental analysis Y = 44%.

### Example (reference) 6

### Attaching of ticarcillin to chitosan in the field of microwaves according to method 2 with the use of HBTU, HOBT and DIPEA.

2.27 g of chitosan suspended in 5 ml of DMF was placed in a reaction vessel for solid state peptide synthesis and subjected to the action of microwaves (t=5 minutes, P=25 W, T=75 °C). 2.14 g HBTU, 1.29 g HOBT and 1.98 ml DIPEA in 5 ml of DMF was added to the reaction vessel. The resulting mixture of chitosan with the activators was processed with microwaves (t=5 minutes, P=25 W, T=75 °C), thus activating the function groups of the polymer. Next, 0.792 g of ticarcillin dissolved in 5 ml of 1:1 DMF:H₂O mixture was added to the vessel and subjected twice to the action of microwaves (t=5 minutes, P=12 W, T<50 °C). The suspension was transferred to a centrifugation vessel and centrifuged. The supernatant was decanted. The obtained modified biopolymer was suspended three times in fresh portions of DMF, and then centrifuged and decanted. This procedure was repeated with the use of methylene chloride. After these three washes with methylene chloride the precipitation was frozen in liquid nitrogen and lyophilized. The lyophilized precipitate was washed three times with distilled water and lyophilized again. The reaction yield determined by elemental analysis Y = 18%.

### Example 7

### Comparison of capabilities of chitosan and glutathione modified chitosan to bind nickel(II) ions.

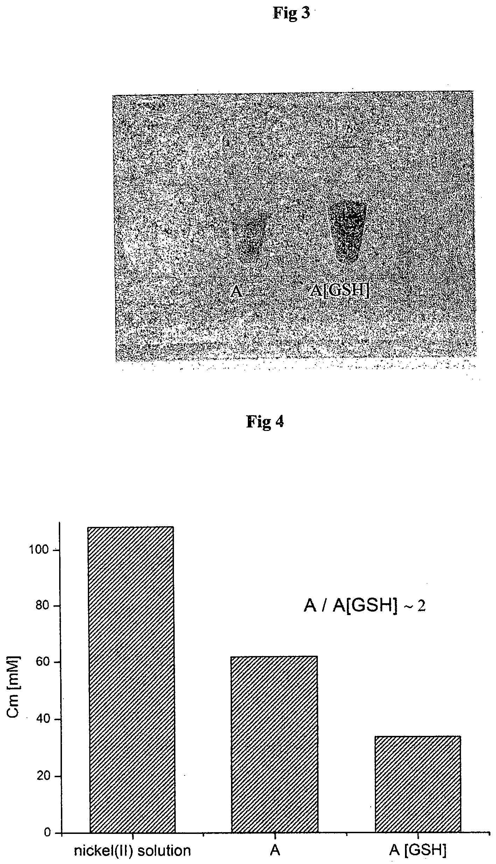

A 35 mg portion of unmodified commercially available chitosan and a 35 mg portion of glutathione modified chitosan obtained according to the invention were dispensed separately into two test tubes, followed by the addition of l ml of 50 mM nickel(II) chloride solution. A discoloration of pale green nickel(II) chloride solution was observed, accompanied by a change of the polymer color, to green for the unmodified chitosan, and to brown for the glutathione modified chitosan (Figure 3). The precipitate settled at the bottom of the test tube, leaving a clear colorless supernatant above. The Ni(II) content in the supernatant was determined by spectrophotometry, using its colored DTT complexes. The change of Ni(II) concentration in solution is illustrated on Figure 4.

### Example 8

### Barrier activity of glutathione modified chitosan against metal ions, in particular nickel(II) ions.

In a vessel composed of three elements, designed for the purpose of this experiment and made with a 3D printer, two layers of dialysis membrane were mounted. The vessel was placed in a 25 ml beaker containing 10 ml of deionized water and a magnetic stirrer. The setup was placed on a magnetic stirrer and used as control experiment. In two further identical vessels 81 mg of commercially available chitosan or 81 mg of glutathione modified chitosan according to the invention was placed between the membrane layers. 0.5 ml of a 100 mM solution of nickel(II) chloride was placed in the inner cylinder of each vessel, and the vessels were placed in 25 ml beakers containing 10 ml of deionized water each. All setups were stirred for 24 hours, thus allowing for diffusion of Ni²⁺ ions across the dialysis membranes and across the layer of chitosan or modified chitosan present between the membranes, respectively. Then, the Ni²⁺ concentrations present in water solutions in each of the beakers were determined. Both polymers demonstrated barrier action with the metal ion concentration detected nearly 8 times tower than that in the control. The results are presented in Table 1. The results in the last column of Table 1 were calculated on the basis of reaction yield Y = 30% given in Example 4.

**Table 1: Results of spectrophotometric assay for concentrations of Ni²⁺ ions in solution.**

| | Test no. | Absorption at 465 nm | Ni²⁺ concentration in solution [mM] | Ni²⁺ in solution vs. control | Ni²⁺ per polymer unit [mol/mol] |
|---|---|---|---|---|---|
| Control | 1 | 0.62 | 4.8 | 100% | 0 |
| | 2 | 0.62 | 4.8 | 100% | 0 |
| Biopolymer (0.455 mmol) | 1 | 0.03 | 0.6 | 13% | 0.092 |
| | 2 | 0.03 | 0.6 | 13% | 0.092 |
| Modified biopolymer (0.306 mmol) | 1 | 0.07 | 0.8 | 17% | 0.131 |
| | 2 | 0.07 | 0.8 | 17% | 0.131 |
| Blank | 1 | 0.00 | 0.00 | 0% | 0 |

The above Table 1 clearly shows that the presence of the chitosan derivative causes a significant reduction of diffusion of Ni²⁺ ions to solution and the chitosan derivative is over 40% more potent than chitosan itself in capturing Ni²⁺. A cosmetic composition containing the chitosan derivative according to the invention as active component has analogous properties, limiting the access of sensitizing ions after placing the composition on the skin.

### Examples of industrial applications of the invention

A use of a new agent (glutathione modified chitosan) provides for effective and simple recovery of metals from water solutions. The use of any desired modifier molecule of chitosan provides an opportunity for controlling the metal chelation properties of the biopolymer, while preserving its biocompatibility and nontoxicity.

The use of chitosan or other biocompatible biopolymer susceptible for attaching modifier molecules, such as of antibiotics used for treatment of dermatitis provides a basis for obtaining new materials for dermatological use.

Peptide LL 37 used broadlly in the cosmetic industry as antimicrobial agent, lactobionic acid helpful in wound healing and combating juvenile acne, and p-aminobenzoic acid used for UVB photoprotection can be listed as such modifiers.

## Claims

1. A chitosan derivative, wherein said chitosan is modified with glutathione with the formation of a peptide bond, to form chitathione, a compound having peptide bonds between amine groups of units of poly(2-deoxy-2-aminoglucose) and available carboxyl groups of glutathione, for use in preventing symptoms of skin allergy caused by the contact with metals, especially heavy metals, in particular nickel.

2. The composition comprising chitosan derivative, wherein said chitosan is modified with glutathione with the formation of a peptide bond, to form chitathione, a compound having peptide bonds between amine groups of units of poly(2-deoxy-2-aminoglucose) and available carboxyl groups of glutathione for use in preventing symptoms of allergy caused by heavy metals, preferably palladium, cobalt, chromium, gold and nickel, especially caused by the contact of skin with nickel.

## Patentansprüche

1. Ein Chitosanderivat, wobei das Chitosan mit Glutathion unter Bildung einer Peptidbindung modifiziert ist, um Chitathion zu bilden, eine Verbindung mit Peptidbindungen zwischen Aminogruppen von Poly(2-desoxy-2-aminoglukose)- Einheiten und verfügbaren Carboxylgruppen von Glutathion, für die Verwendung zur Verhinderung von Symptomen einer Hautallergie, verursacht durch den Kontakt mit Metallen, insbesondere Schwermetallen, insbesondere Nickel.

2. Die Zusammensetzung, die ein Chitosanderivat beinhalten, wobei das Chitosan mit Glutathion unter Bildung einer Peptidbindung modifiziert ist, um Chitathion zu bilden - eine Verbindung mit Peptidbindungen zwischen Aminogruppen von Poly(2-desoxy-2-aminoglukose)-Einheiten und verfügbaren Carboxyl-Gruppen von Glutathion, zur Verwendung bei der Vorbeugung von Allergiesymptomen, verursacht durch Schwermetalle, vorzugsweise Palladium, Kobalt, Chrom, Gold und Nickel, verursacht insbesondere durch den Kontakt der Haut mit Nickel.

## Revendications

1. Dérivé de chitosane dans lequel le chitosane est modifié avec du glutathion pour former une liaison peptidique, pour former de la chitathione, un composé ayant des liaisons peptidiques entre les groupes amino des unités poly(2-désoxy-2-aminoglucose) et les groupes carboxyle disponibles du glutathion, destiné à être utilisé dans la prévention des symptômes d'allergie cutanée provoquée par le contact avec des métaux, en particulier des métaux lourds, notamment le nickel.

2. Une composition comprenant un dérivé de chitosane dans laquelle le chitosane est modifié avec du glutathion pour former une liaison peptidique afin de former de la chitathione, un composé ayant des liaisons peptidiques entre les groupes amino des unités poly(2-désoxy-2-aminoglucose) et les groupes carboxyle disponibles du glutathion, destiné à être utilisé dans la prévention des symptômes d'allergie causés par les métaux lourds, de préférence le palladium, le cobalt, le chrome, l'or et le nickel, en particulier ceux causés par le contact cutané avec le nickel.
